# EUROPÄISCHE PATENTSCHRIFT

(11) **EP 4 000 541 B1**
(45) Veröffentlichungstag und Bekanntmachung des Hinweises auf die Patenterteilung: **03.05.2023**
(21) Anmeldenummer: 20207244.3
(22) Anmeldetag: 12.11.2020
(51) Int. Cl.: A61B 17/285, A61B 18/14

(54) **CHIRURGISCHES INSTRUMENT**
SURGICAL INSTRUMENT
INSTRUMENT CHIRURGICAL

(43) Veröffentlichungstag der Anmeldung: 25.05.2022
(73) Patentinhaber: Erbe Elektromedizin GmbH, 72072 Tübingen (DE)
(72) Erfinder: BOB Felix,, 72108 Rottenburg (DE)
(74) Vertreter: Rüger Abel Patentanwälte PartGmbB

(56) Entgegenhaltungen:
- EP-A1- 2 436 327
- US-A1- 2016 066 981
- US-A1- 2016 157 922
- US-A1- 2018 325 580

## Beschreibung

Die Erfindung betrifft ein chirurgisches Instrument aufweisend eine erste Branche und eine zweite Branche, die über ein Gelenk schwenkbar aneinander gelagert sind. Die beiden Branchen können zwischen einer Offenstellung und einer Schließstellung bewegt werden. Jede Branche kann ein Maulteil aufweisen. Vorzugsweise weist jede Branche ein unbeweglich mit dem Maulteil verbundenes Schaftteil auf, wobei die Schaftteile der beiden Branchen zu deren Betätigung dienen. Das chirurgische Instrument kann beispielsweise zangenartig oder scherenartig ausgestaltet sein.

Außerdem weist das chirurgische Instrument ein Werkzeug auf, beispielsweise ein Messer, das in oder an der ersten Branche in einer Längsrichtung geführt bewegbar gelagert ist. Die erste Branche kann hierfür einen Werkzeugkanal bzw. Messerkanal aufweisen.

Bei solchen chirurgischen Instrumenten ist es wünschenswert, eine ungewollte Werkzeugbetätigung zu vermeiden. Insbesondere ist es gewünscht, das Ausfahren des Werkzeugs aus dem Werkzeugkanal zu erschweren oder zu verhindern, wenn die Branchen geöffnet sind.

EP 2 436 327 A1 beschreibt ein chirurgisches Instrument mit zwei Branchen und einem bewegbar gelagerten Messer. Bei einigen Ausführungsbeispielen ist ein Blockierelement vorhanden, das in der Offenstellung der Branchen das Bewegen eines Betätigungselements blockiert, das zum Verschieben des Messers geschwenkt werden kann. In einer Freigabestellung des Blockierelements wird das Schwenken des Betätigungselements zugelassen, so dass das Messer in Längsrichtung ausgefahren werden kann. Bei einer anderen Ausführungsform wird das Schwenken des Betätigungselements bei geöffneten Branchen erschwert, indem ein Rückstellelement in der Offenstellung der Branchen gespannt wird, so dass eine deutlich erhöhte Kraft notwendig wäre, um das Betätigungselement zu schwenken. Außerdem ist noch ein Ausführungsbeispiel mit linear bewegbarem Betätigungselement vorhanden, bei dem die Bewegungskopplung mit dem Messer nur bei geschlossenen Branchen hergestellt ist.

US 2017/0128120 A1 beschreibt ein chirurgisches Instrument mit zwei Branchen und einem linear bewegbaren Betätigungselement zum Verschieben eines Messers. Das Betätigungselement weist eine erste Zahnstange auf, die in Eingriff mit einem Zahnrad steht. Das Betätigungselement und das Zahnrad sind an der zweiten Branche angeordnet. An der ersten Branche ist das Messer und eine fest mit dem Messer verbundene zweite Zahnstange angeordnet. Das Zahnrad und die zweite Zahnstange sind in der Offenstellung außer Eingriff und lediglich in der Schließstellung in Eingriff, so dass nur in der Schließstellung ein Verschieben des Messers bewirkt wird, wenn das Betätigungselement bewegt wird.

US 2016/0302844 A1 offenbart ein chirurgisches Instrument mit zwei Branchen. Ein linear bewegbares Betätigungselement zum Bewegen eines Messers wird über eine Kopplungseinrichtung mit dem Messer gekoppelt, wenn sich die Branchen in der Schließstellung befinden. Dieses Prinzip entspricht einem Ausführungsbeispiel der EP 2 436 327 A1 mit linear bewegbarem Betätigungselement.

Ein linear bewegbares Betätigungselement ist aus ergonomischen Gründen häufig nicht gewünscht. Außerdem wird am Instrument relativ viel Platz benötigt, um den linearen Bewegungsweg für das Betätigungselement bereit zu stellen. Die Schaftteile der Branchen werden dadurch häufig sehr lang. Dies wiederum hat Auswirkungen auf den Öffnungswinkel der Maulteile der Branchen, wenn die Schaftteile am proximalen Ende voneinander weg bewegt werden.

Es kann daher als Aufgabe der vorliegenden Erfindung angesehen werden, ein chirurgisches Instrument mit einem schwenkbaren Betätigungselement zu schaffen, das bei einfachem Aufbau eine hohe Arbeitssicherheit gewährleistet.

Diese Aufgabe wird durch ein chirurgisches Instrument mit den Merkmalen des Patentanspruches 1 gelöst.

Das erfindungsgemäße chirurgische Instrument weist eine erste Branche und eine zweite Branche auf, die gelenkig aneinander gelagert sind. Jede Branche hat ein Maulteil mit einer Gewebekontaktfläche im distalen Endbereich. Jedes Maulteil ist mit einem Schaftteil der jeweiligen Branche verbunden. Im Bereich des Gelenks der Branchen gehen das Schaftteil und das Maulteil ineinander über bzw. sind miteinander verbunden.

An der ersten Branche ist ein Werkzeug in einer Längsrichtung geführt bewegbar gelagert. An der ersten Branche ist außerdem ein Betätigungselement und ein Antriebselement schwenkbar um eine Schwenkachse gelagert. Das Antriebselement ist mit dem Werkzeug bewegungsgekoppelt, beispielsweise über eine Übertragungseinrichtung, wie etwa ein Getriebe, insbesondere ein Hebelgetriebe.

Das Instrument weist außerdem eine Kopplungseinrichtung mit einem Kopplungselement auf. Das Kopplungselement ist zwischen einer Kopplungsstellung und einer Entkopplungsstellung bewegbar gelagert. Das Kopplungselement stellt in der Kopplungsstellung eine Drehkopplung bzw. eine drehfeste Verbindung zwischen dem Betätigungselement und dem Antriebselement her. Bei einer Schwenkbewegung des Betätigungselements um die Schwenkachse schwenkt daher auch das Antriebselement um die Schwenkachse. In der Entkopplungsstellung ist diese Drehkopplung bzw. drehfeste Verbindung aufgehoben und eine Relativdrehung zwischen dem Betätigungselement und dem Antriebselement um die Schwenkachse ist ermöglicht. Wenn in der Entkopplungsstellung das Betätigungselement um die Schwenkachse geschwenkt wird, bleibt das Betätigungselement davon unbeeinflusst. Das Antriebselement kann unabhängig vom Schwenken des Betätigungselements in seiner Ausgangslage bleiben, wenn das Kopplungselement die Entkopplungsstellung einnimmt.

Das Kopplungselement ist gemeinsam mit dem Betätigungselement um die Schwenkachse schwenkbar gelagert. Das Kopplungselement ist am Betätigungselement bewegbar angeordnet. Insbesondere ist das Herstellen der Drehkopplung bzw. das Bewegen des Kopplungselements in die Kopplungsstellung nur möglich, wenn sich sowohl das Betätigungselement, als auch das Antriebselement in ihrer jeweiligen Ausgangslage befinden.

In der Kopplungsstellung wird bevorzugt eine Drehkopplung in beide Drehrichtungen bzw. in beide Drehsinne um die Schwenkachse hergestellt.

Das Kopplungselement kann manuell zwischen der Kopplungsstellung und der Entkopplungsstellung bewegt werden, beispielsweise durch unmittelbare Betätigung mit einem Finger. Alternativ oder zusätzlich ist das Kopplungselement über die zweite Branche betätigbar bzw. bewegbar. Dazu ist das chirurgische Instrument insbesondere derart eingerichtet, dass das Kopplungselement aus der Entkopplungsstellung in die Kopplungsstellung bewegt wird, wenn die Branchen relativ zueinander in die Schließstellung geschwenkt werden. Bei dieser Bewegung in die Schließstellung gelangt die zweite Branche mittelbar oder unmittelbar in Kontakt mit dem Kopplungselement und bewegt das Kopplungselement in die Kopplungsstellung.

Das Kopplungselement wird vorzugsweise aus der Kopplungsstellung in die Entkopplungsstellung bewegt, wenn die Branchen aus der Schließstellung in die Offenstellung bewegt werden und wenn sich das Betätigungselement und das Antriebselement in ihrer jeweiligen Ausgangslage befinden. Wenn das Kopplungselement in der Kopplungsstellung eine drehfeste Kupplung zwischen dem Betätigungselement und dem Antriebselement herstellt wird das Kopplungselement bevorzugt kraftschlüssig und/oder formschlüssig in der Kopplungsstellung gehalten, auch wenn die Branchen von der Schließstellung in die Offenstellung bewegt werden.

Bei allen Ausführungsbeispielen ist sichergestellt, dass das Werkzeug, beispielsweise ein Messer, nicht versehentlich betätigt wird. In der Entkopplungsstellung kann das Betätigungselement geschwenkt werden, ohne dass dadurch eine Werkzeugbewegung in Längsrichtung stattfindet. Wird das Kopplungselement hingegen in die Kopplungsstellung bewegt, führt das Betätigen des Betätigungselements zu einer Schwenkbewegung des Antriebselements um die Schwenkachse, das wiederum bewegungsgekoppelt ist mit dem Werkzeug und auf diese Weise eine Werkzeugbewegung veranlasst.

Bei einer bevorzugten Ausführungsform weist das Antriebselement eine Kopplungsaussparung auf. In der Kopplungsstellung greift das Kopplungselement in die Kopplungsaussparung ein. Dadurch ist eine drehfeste Verbindung zwischen dem Antriebselement und dem Betätigungselement geschaffen. In der Entkopplungsstellung befindet sich das Kopplungselement vollständig außerhalb der Kopplungsaussparung. Die Kopplungsaussparung ist vorzugsweise durch zwei sich in Umfangsrichtung um die Schwenkachse mit Abstand gegenüberliegende Flanken begrenzt. Eine derart ausgeführte Kopplungseinrichtung lässt sich mit wenigen Bauteilen auf einfache Weise realisieren.

Bei einer bevorzugten Ausführungsform des chirurgischen Instruments kann das Kopplungselement zwischen der Kopplungsstellung und der Entkopplungsstellung verschiebbar bzw. linear bewegbar sein. Bei einer alternativen Ausführungsform kann das Kopplungselement zwischen der Kopplungsstellung und der Entkopplungsstellung schwenkbar sein. Alternativ dazu sind auch überlagerte Linearbewegungen und Schwenkbewegungen realisierbar. Unabhängig von der Bewegungsbahn des Kopplungselements verläuft diese zumindest in ihrem Endabschnitt vor dem Erreichen der Kopplungsstellung im Wesentlichen rechtwinklig oder radial zur Schwenkachse.

Es ist vorteilhaft, wenn eine Vorspanneinrichtung vorhanden ist. Die Vorspanneinrichtung ist dazu eingerichtet, eine Vorspannkraft auf das Kopplungselement zu erzeugen. Mittels der Vorspannkraft wird das Kopplungselement in die Entkopplungsstellung gedrängt. Die Vorspannkraft kann eine Druckkraft und/oder Zugkraft sein. Beispielsweise kann die Vorspanneinrichtung zur Erzeugung der Vorspannkraft ein Federelement aufweisen. Ein einziges Federelement ist ausreichend.

Bei einem Ausführungsbeispiel können das Federelement und das Kopplungselement unmittelbar miteinander verbunden sein angeordnet sein. Die Verbindung kann als lösbare Verbindung oder als unlösbare Verbindung ausgebildet sein. Es ist beispielsweise möglich, das Kopplungselement und das Federelement durch eine stoffschlüssige Verbindung und/oder eine Haftverbindung miteinander zu verbinden. Beispielsweise kann das Federelement mittels eines Klebstoffes, durch Umspritzen oder Umgießen mit dem Kopplungselement verbunden sein.

Zusätzlich oder alternativ kann das Federelement lösbar oder unlösbar am Betätigungselement angeordnet sein, beispielsweise durch eine stoffschlüssige Verbindung und/oder eine Haftverbindung. Beispielsweise kann das Federelement mittels eines Klebstoffes, durch Umspritzen oder Umgießen mit dem Betätigungselement verbunden sein.

Bei einer bevorzugten Ausführungsform können das Federelement und das Kopplungselement integral ausgebildet sein. Das Federelement und das Kopplungselement können ohne Naht- und Fügestelle ineinander übergehen. Bei dieser Ausgestaltung ist es vorteilhaft, wenn das Federelement und das Kopplungselement beispielsweise aus Metall oder einer metallischen Legierung bestehen. Sie können durch Trennen und/oder Umformen aus einem Blechteil oder aus einem Draht hergestellt sein.

Es ist bevorzugt, wenn das Federelement eine Biegefeder oder eine Blattfeder ist. Die Biegefeder oder Blattfeder kann eine Vorspannkraft rechtwinklig oder radial zur Schwenkachse erzeugen. Die Biegefeder kann sich beispielsweise bogenförmig um die Schwenkachse erstrecken insbesondere einem Winkelbereich von mindestens 90° oder mindestens 120° oder mindestens 180°. Die Blattfeder kann sich geradlinig oder gekrümmt von einem Befestigungsende zu einem freien Ende erstrecken. erstrecken. Das Befestigungsende der Blattfeder kann einen größeren Abstand zu der Schwenkachse aufweisen als das freie Ende.

Alternativ dazu kann das Federelement eine Schraubenfeder sein. Die Schraubenfeder kann beispielsweise rechtwinklig oder radial zur Schwenkachse ausgerichtet sein. Bei allen Ausführungsbeispielen des Federelements stützt sich das Federelement einerseits am Betätigungselement und andererseits am Kopplungselement ab.

Bei einer vorteilhaften Ausführungsform weist das chirurgische Instrument eine Rückstelleinrichtung auf. Die Rückstelleinrichtung ist dazu eingerichtet, das Betätigungselement und/oder das Antriebselement in einer Ausgangslage zu drängen. Hierzu kann die Rückstelleinrichtung ein erstes Rückstellelement für das Antriebselement und/oder ein zweites Rückstellelement für das Betätigungselement aufweisen. Es kann vorteilhaft sein, wenn das erste Rückstellelement durch ein elastisch verformbares Übertragungsglied gebildet ist, das zwischen dem Antriebselement und dem Werkzeug angeordnet ist, beispielsweise ein elastisch verformbares Pleuel.

Wenn lediglich ein einziges Rückstellelement für das Betätigungselement vorhanden ist, wirkt die Rückstellkraft in der Entkopplungsstellung des Kopplungselements nicht auf das Antriebselement. Um ein versehentliches Bewegen des Werkzeugs zu vermeiden, kann das Antriebselement in der Entkopplungsstellung des Kopplungselements gegen ein Schwenken um die Schwenkachse gesichert sein. Dazu kann beispielsweise das Kopplungselement in der Entkopplungsstellung verwendet werden. Vorzugsweise ist das Kopplungselement in der Entkopplungsstellung mit der Branche und dem Antriebselement in Eingriff, nicht jedoch mit dem Betätigungselement.

Vorteilhafte Ausgestaltungen der Erfindung ergeben sich aus den abhängigen Ansprüchen, der Beschreibung und den Zeichnungen. Nachfolgend werden bevorzugte Ausführungsbeispiele der Erfindung anhand der beigefügten Zeichnungen im Einzelnen erläutert. Die Zeichnungen zeigen:
Figuren 1-3 eine schematische Seitenansicht eines chirurgischen Instruments, insbesondere eines elektrochirurgischen Instruments mit einem bewegbaren Werkzeug und einer zum Bewegen des Werkzeugs vorhandenen Betätigungsvorrichtung in unterschiedlichen Zuständen der Betätigungsvorrichtung,
Figur 4 ein Ausführungsbeispiel einer Betätigungsvorrichtung für das elektrochirurgische Instrument aus den Figuren 1-3 in einer schematischen Seitenansicht, wobei sich eine Kopplungseinrichtung der Betätigungsvorrichtung in einem Entkopplungszustand befindet,
Figur 5 die Betätigungsvorrichtung aus Figur 4, wobei sich die Kopplungseinrichtung in einem Kopplungszustand befindet,
Figur 6 ein Ausführungsbeispiel einer Betätigungsvorrichtung für das elektrochirurgische Instrument aus den Figuren 1-3 in einer schematischen Seitenansicht, wobei die Kopplungseinrichtung ein bewegbares Kopplungselement aufweist, das sich in einer Entkopplungsstellung befindet,
Figur 7 die Betätigungsvorrichtung aus Figur 6, wobei sich das Kopplungselement in einer Kopplungsstellung befindet,
Figur 8 ein Ausführungsbeispiel einer Betätigungsvorrichtung für das elektrochirurgische Instrument aus den Figuren 1-3 in einer schematischen Seitenansicht wobei die Kopplungseinrichtung ein bewegbares Kopplungselement aufweist, das sich in einer Entkopplungsstellung befindet,
Figur 9 die Betätigungsvorrichtung aus Figur 8, wobei sich das Kopplungselement in einer Kopplungsstellung befindet,
Figur 10 ein Ausführungsbeispiel einer Betätigungsvorrichtung für das elektrochirurgische Instrument aus den Figuren 1-3 in einer schematischen Seitenansicht wobei die Kopplungseinrichtung ein bewegbares Kopplungselement aufweist, das sich in einer Entkopplungsstellung befindet,
Figur 11 die Betätigungsvorrichtung aus Figur 10, wobei sich das Kopplungselement in einer Kopplungsstellung befindet,
Figur 12 ein Ausführungsbeispiel einer Betätigungsvorrichtung für das elektrochirurgische Instrument aus den Figuren 1-3 in einer schematischen Seitenansicht wobei die Kopplungseinrichtung ein bewegbares Kopplungselement aufweist, das sich in einer Entkopplungsstellung befindet,
Figur 13 die Betätigungsvorrichtung aus Figur 12, wobei sich das Kopplungselement in einer Kopplungsstellung befindet,
Figur 14 die Betätigungsvorrichtung aus den Figuren 12 und 13, wobei sich das Kopplungselement in einer Entkopplungsstellung befindet und das Betätigungselement der Betätigungsvorrichtung aus einer Ausgangslage heraus geschwenkt ist, und
Figur 15 ein weiteres Ausführungsbeispiel der Betätigungsvorrichtung basierend auf dem Ausführungsbeispiel der Figuren 8 und 9, wobei sich das Kopplungselement in einer Entkopplungsstellung und das Betätigungselement der Betätigungsvorrichtung in der Ausgangslage befinden.

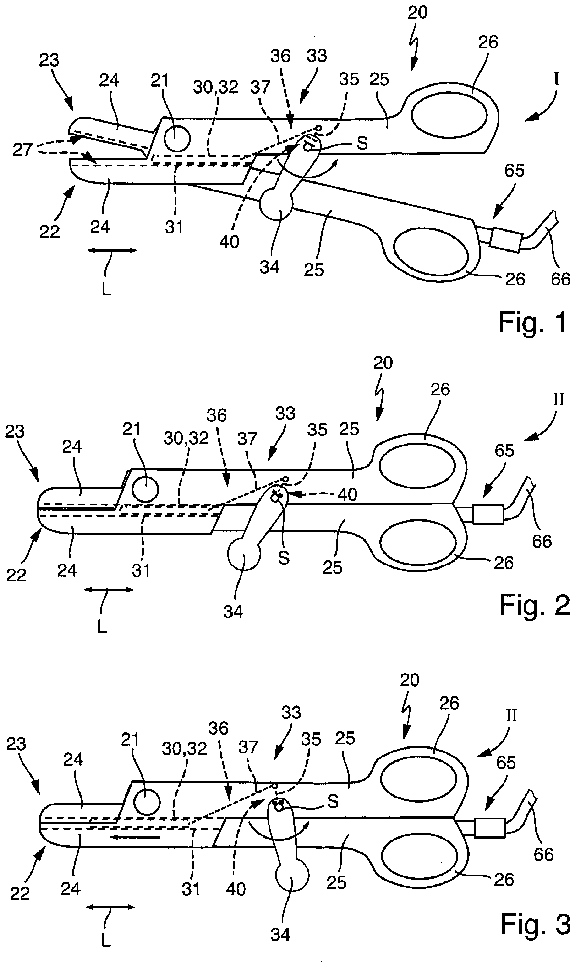

In den Figuren 1-3 ist schematisch ein Ausführungsbeispiel eines chirurgischen Instruments 20 veranschaulicht. Das chirurgische Instrument 20 ist insbesondere als Mehrweginstrument ausgebildet. Alternativ hierzu könnte das chirurgische Instrument auch als Einweginstrument realisiert sein.

Wie veranschaulicht, ist das chirurgische Instrument 20 beim Ausführungsbeispiel nach Art einer Schere oder Zange ausgebildet. Es weist ein Gelenk 21 auf, mittels dem eine erste Branche 22 und eine zweite Branche 22 schwenkbar aneinander gelagert sind. Jede Branche 22, 23 weist im distalen Bereich ein Maulteil 24 auf. Jede Branche 22, 23 weist außerdem ein Schaftteil 25 auf, wobei die beiden Schaftteile 25 zur Bedienung der Maulteile 24 eingerichtet sind. Hierzu hat jedes Schaftteil 25 ein Griffstück 26 am proximalen Ende. Die Griffstücke 26 der beiden Branchen 22, 23 können voneinander weg bzw. aufeinander zu bewegt werden, wodurch entsprechend die Maulteile 24 voneinander weg bzw. aufeinander zu bewegt werden können. Das Instrument 20 arbeitet ähnlich wie eine Zange oder eine Schere.

Jedes Maulteil 24 weist eine Gewebekontaktfläche 27 auf. Die beiden Gewebekontaktflächen 27 sind einander zugewandt und dazu eingerichtet, biologisches Gewebe dazwischen zu halten bzw. einzuklemmen. In einer Offenstellung I sind die beiden Schaftteile 25 und die beiden Maulteile 24 um das Gelenk 21 voneinander weg geschwenkt (Figur 1). In einer Schließstellung II sind die Schaftteile 25 bzw. die Maulteile 25 und/oder die Gewebekontaktflächen 27 im Wesentlichen parallel zueinander ausgerichtet. Die Gewebekontaktflächen 27 weisen in der Schließstellung II einen minimalen Abstand voneinander auf. In der Schließstellung II kann biologisches Gewebe zwischen den Maulteilen 24 bzw. den Gewebekontaktflächen 27 eingeklemmt oder gehalten werden.

Das chirurgische Instrument weist außerdem ein Werkzeug 30 auf, das in einem Werkzeugkanal 31 in oder an der ersten Branche 22 in einer Längsrichtung L bewegbar bzw. verschiebbar gelagert ist. Bei dem Werkzeug 30 handelt es sich insbesondere um ein Messer 32, das in Längsrichtung L zwischen einer ausgefahrenen Stellung (Figur 3) und einer eingefahrenen Stellung (Figuren 1 und 2) bewegt werden kann. In der ausgefahrenen Stellung kann zwischen den Gewebekontaktflächen 27 bzw. zwischen den Maulteilen 24 eingeklemmtes Gewebe geschnitten oder durchtrennt werden.

Zur Bewegung des Werkzeugs 30 und beispielsgemäß des Messers 32 in Längsrichtung L weist das Instrument 20 eine Betätigungsvorrichtung 33 auf. Die Betätigungsvorrichtung 33 hat ein um eine Schwenkachse S an der ersten Branche 22 schwenkbar gelagertes Betätigungselement 34. Die Schwenkachse S erstreckt sich rechtwinklig zur Längsrichtung L im Wesentlichen parallel zu einer durch das Gelenk 21 definierten Gelenkachse. Das Betätigungselement 34 kann parallel zur Schwenkachse S neben den beiden Branchen 22, 23 angeordnet sein. Ausgehend von der Schwenkachse S erstreckt sich das Betätigungselement 34 zu einem freien Ende hin, das durch eine Bedienperson mit der Hand bzw. einem Finger bedienbar ist, um das Betätigungselement 34 um die Schwenkachse S zu schwenken.

Die Betätigungsvorrichtung 33 hat außerdem ein Antriebselement 35, das schwenkbar um die Schwenkachse S gelagert ist. In den Figuren 1-3 ist das Antriebselement 35 durch einen Arm bzw. Hebel lediglich schematisch veranschaulicht.

Das Antriebselement 35 ist mittels einer Übertragungseinrichtung 36 mit dem Werkzeug 30 und beispielsgemäß dem Messer 32 bewegungsgekoppelt. Die Übertragungseinrichtung 36 weist wenigstens ein Übertragungsglied 37 auf und ist beispielsgemäß durch ein einziges Übertragungsglied 37 gebildet, das als Pleuel ausgeführt ist und das Werkzeug 30 mit dem Antriebselement 35 verbindet bzw. koppelt. Über das Übertragungsglied 37 wird die Schwenkbewegung des Antriebselements 35 um die Schwenkachse S in eine lineare Bewegung des Werkzeugs 30 in Längsrichtung L umgesetzt. Dazu kann das Übertragungsglied 37 bei einem Ausführungsbeispiel gelenkig bzw. drehbar mit dem Antriebselement 35 und/oder dem Werkzeug 30 verbunden sein.

Stark schematisiert ist in den Figuren 1-3 außerdem eine Kopplungseinrichtung 40 gezeigt. Die Kopplungseinrichtung 40 kann einen Entkopplungszustand (Figur 1) und einen Kopplungszustand (Figuren 2 und 3) annehmen. In der Entkopplungsstellung kann das Betätigungselement 34 um die Schwenkachse S geschwenkt werden, ohne dabei die Schwenkstellung des Antriebselements 35 um die Schwenkachse S zu beeinflussen. Im Kopplungszustand sind das Betätigungselement 34 und das Antriebselement 35 derart drehfest miteinander verbunden, dass ein Schwenken des Betätigungselements 34 um die Schwenkachse S zu einer Schwenkbewegung des Antriebselements 35 um die Schwenkachse S führt.

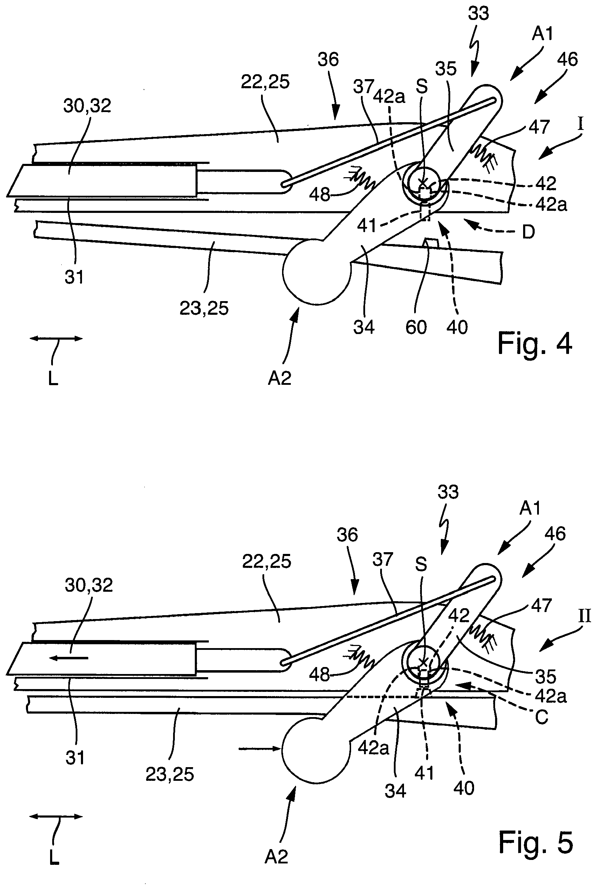

Ein Ausführungsbeispiel der Betätigungsvorrichtung 33 ist anhand einer Prinzipdarstellung schematisch in den Figuren 4 und 5 veranschaulicht. Die Kopplungseinrichtung 40 weist beispielsgemäß ein bewegbares Kopplungselement 41 auf, das zwischen einer Entkopplungsstellung D (Figur 4) und einer Kopplungsstellung C (Figur 5) bewegt werden kann. In der Kopplungsstellung C befindet sich die Kopplungseinrichtung 40 im Kopplungszustand und in der Entkopplungsstellung D befindet sich die Kopplungseinrichtung 40 im Entkopplungszustand.

Wie es schematisch in den Figuren 4 und 5 veranschaulicht ist, weist das Antriebselement 35 eine Kopplungsaussparung 42 auf, die beispielsgemäß rechtwinklig oder radial zur Schwenkachse S ausgerichtet ist. Das Kopplungselement 41 ist am Betätigungselement 34 angeordnet. Auf der dem Betätigungselement 34 zugewandten Seite ist die Kopplungsaussparung 42 offen. In der Entkopplungsstellung D befindet sich das Kopplungselement 41 vollständig außerhalb der Kopplungsaussparung 42. In der Kopplungsstellung C greift das Kopplungselement 41 in die Kopplungsaussparung 42 ein und stellt dadurch die Drehkopplung bzw. drehfeste Verbindung zwischen dem Betätigungselement 34 und dem Antriebselement 35 her. Die Kopplungsaussparung 42 ist vorzugsweise durch zwei sich in Umfangsrichtung um die Schwenkachse S mit Abstand gegenüberliegende Flanken 42a begrenzt.

Das Kopplungselement 41 ist vorzugsweise rechtwinklig oder radial zur Schwenkachse S linear bewegbar und/oder schwenkbar gelagert. Zumindest der Endabschnitt der Bewegungsbahn des Kopplungselements 41, der sich an die Kopplungsstellung C anschließt, kann rechtwinklig zur Schwenkachse S ausgerichtet sein.

In der Entkopplungsstellung D des Kopplungselements 41 können das Betätigungselement 34 und das Antriebselement 35 unabhängig voneinander um die Schwenkachse S bewegt werden. Dies bedeutet, dass ein Schwenken des Betätigungselements 34 um die Schwenkachse S die Schwenklage des Antriebselements 35 nicht verändert. Nimmt das Kopplungselement 41 hingegen die Kopplungsstellung C ein, führt ein Schwenken des Betätigungselements 34 zu einer Schwenkbewegung des Antriebselements 35, die wiederum über das Übertragungsglied 37 in eine lineare Bewegung des Werkzeugs 30 überführt wird. Dadurch kann das Werkzeug 30 ausgefahren werden und in den Bereich der Maulteile 24 eingreifen. Beispielsweise kann das Messer 32 zwischen den Maulteilen 24 gehaltenes biologisches Gewebe schneiden bzw. durchtrennen.

Wie es in den Figuren 4 und 5 schematisch ebenfalls veranschaulicht ist, weist das Instrument 20 eine Rückstelleinrichtung 46 auf. Die Rückstelleinrichtung 46 ist dazu eingerichtet, das Betätigungselement 34 und das Antriebselement 35 in eine jeweilige Ausgangslage A1 bzw. A2 zu drängen. Dazu weist die Rückstelleinrichtung 46 beispielsweise ein erstes Rückstellelement 47 auf, das mit dem Antriebselement 35 verbunden ist sowie ein zweites Rückstellelement 48, das mit dem Betätigungselement 34 verbunden ist. Die Rückstellelemente 47, 48 sind jeweils dazu eingerichtet, eine Rückstellkraft zu erzeugen und können als federelastische Rückstellelemente 47, 48 ausgebildet sein. Die Rückstellelemente 47, 48 können beispielsweise durch Blattfedern, Schraubenfedern, Spiralfedern oder dergleichen gebildet sein. Die Rückstellelemente 47, 48 stützen sich am Schaftteil 25 der ersten Branche 22 ab und erzeugen somit die Rückstellkraft zwischen der ersten Branche 22 einerseits und dem Betätigungselement 34 bzw. dem Antriebselement 35 andererseits.

Beim Ausführungsbeispiel drängt das erste Rückstellelement 47 das Antriebselement 35 in eine erste Ausgangslage A1 und das zweite Rückstellelement 48 drängt das Betätigungselement 34 in eine zweite Ausgangslage A2. Wenn sich sowohl das Betätigungselement 34 als auch das Antriebselement 35 in ihrer jeweiligen Ausgangslage A1, A2 befinden, fluchtet das Kopplungselement 41 am Betätigungselement 34 mit der Kopplungsaussparung 42 am Antriebselement 35. Das Herstellen der Kopplungsstellung C ist möglich. Wird das Betätigungselement 34 aus seiner zweiten Ausgangslage A2 heraus geschwenkt, solange sich das Kopplungselement 41 in der Entkopplungsstellung D befindet, ist ein Herstellen der Drehkopplung nicht mehr möglich. Das Betätigungselement 34 muss zunächst in die zweite Ausgangslage A2 zurück bewegt werden.

Bei den hier veranschaulichten bevorzugten Ausführungsbeispielen wird das Kopplungselement 41 durch die zweite Branche 23 und beispielsgemäß das Schaftteil 25 der zweiten Branche 23 betätigt bzw. von der Entkopplungsstellung D in die Kopplungsstellung C bewegt, wie es schematisch in Figur 5 zu erkennen ist. In der Schließstellung II steht die zweite Branche 23 in Kontakt mit dem Kopplungselement 41 und bewegt das Kopplungselement 41 in die Kopplungsaussparung 42, so dass die Kopplungsstellung C eingenommen wird. Diese Verschiebung ist dann möglich, wenn sich das Betätigungselement 34 und das Antriebselement 35 in ihrer jeweiligen Ausgangslage A1, A2 befinden.

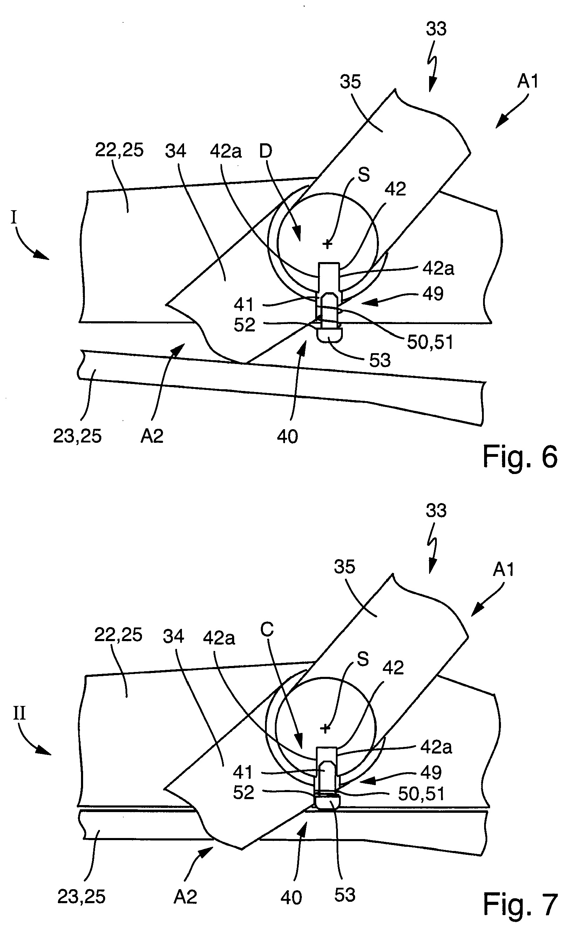

In den Figuren 6-14 sind unterschiedliche Ausführungsformen der Betätigungsvorrichtung 33 veranschaulicht. Bei allen Ausführungsbeispielen ist eine Vorspanneinrichtung 49 vorhanden, die dazu eingerichtet ist, das Kopplungselement 41 in die Entkopplungsstellung D zu drängen. Hierzu erzeugt die Vorspanneinrichtung 49 eine Vorspannkraft in Richtung der Entkopplungsstellung D auf das Kopplungselement 41. Die Vorspannkraft kann eine Zugkraft und/oder eine Drückkraft sein. Die Vorspanneinrichtung 49 ist gemeinsam mit dem Kopplungselement 41 am Betätigungselement 34 angeordnet.

Zur Erzeugung der Vorspannkraft weist die Vorspanneinrichtung 49 vorzugsweise ein Federelement 50 auf. Das Federelement 50 kann bei einem Ausführungsbeispiel als Schraubenfeder ausgebildet sein (Figuren 6 und 7). Dabei kann beispielsweise ein Ende der Schraubenfeder mit dem Kopplungselement 41 und das jeweils andere Ende der Schraubenfeder 51 mit dem Betätigungselement 34 verbunden sein. Die Verbindung kann als stoffschlüssige Verbindung und/oder Haftverbindung ausgebildet sein. Zwischen den beiden Enden weist die Schraubenfeder 51 einen sich schrauben- oder helixförmig erstreckenden Federabschnitt auf, der sich beispielsweise um ein zylindrisches Kopplungselement 41 herum winden kann.

Das mit dem Kopplungselement 41 verbundene Ende kann an einer Ringstufe 52 des Kopplungselements 41 anliegen oder dort befestigt sein. Die Ringstufe 52 kann durch einen Übergang von einem der Kopplungsaussparung 42 zugeordneten zylindrischen Abschnitt zu einem radial erweiterten Kopfteil 53 des Kopplungselements 41 gebildet sein. Der Kopfteil 53 ist dem Schaftteil 25 der zweiten Branche 23 zugewandt.

In der Offenstellung des chirurgischen Instruments 20 (Figur 6) sind die Schaftteile 25 der beiden Branchen 22, 23 mit Abstand zueinander angeordnet. Werden die Branchen 22, 23 in die Schließstellung bewegt, nähern sich die Schaftteile 25 an und das Schaftteil 25 der zweiten Branche 23 gelangt in Kontakt mit dem Kopfteil 53 des Kopplungselements 41. Bei einer weiteren Bewegung in Richtung der Schließstellung II wird das Kopplungselement 41 gegen die Vorspannkraft der Vorspanneinrichtung 49 und beispielsgemäß der Schraubenfeder 51 in die Kopplungsaussparung 42 bewegt, so dass die Kopplungsstellung C hergestellt wird. Im Anschluss daran kann durch das Schwenken des Betätigungselements 34 eine Schwenkbewegung des Antriebselements 35 bewirkt und dadurch das Werkzeug 30 bzw. das Messer 32 ausgefahren bzw. auch wieder eingefahren werden.

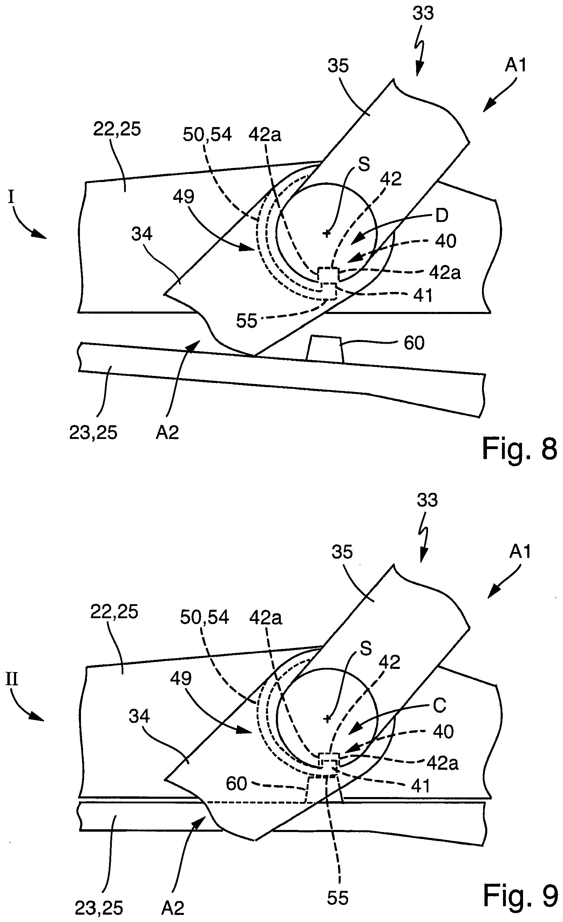

In den Figuren 8 und 9 ist schematisch ein weiteres Ausführungsbeispiel der Betätigungsvorrichtung 33 veranschaulicht. Im Wesentlichen kann auf die Beschreibung gemäß den Figuren 4-7 verwiesen werden. Der wesentliche Unterschied zu der Ausführungsform der Betätigungsvorrichtung gemäß der Figuren 6 und 7 besteht beim Ausführungsbeispiel gemäß der Figuren 8 und 9 darin, dass das Federelement 50 der Vorspanneinrichtung 49 als gekrümmte oder bogenförmige Biegefeder 54 ausgebildet ist. An der Biegefeder 54 ist das Kopplungselement 41 angeordnet. Beim Ausführungsbeispiel ist das Kopplungselement 41 an einem freien Ende 55 der Biegefeder angeordnet. Das Kopplungselement 41 kann unlösbar mit der Biegefeder 54 verbunden sein. Vorzugsweise sind die Biegefeder 54 und das Kopplungselement 41 integral ausgebildet.

Die Biegefeder 54 ist mit Abstand zum Kopplungselement 41 und beispielsgemäß an ihrem entgegengesetzten Befestigungsende am Betätigungselement 34 befestigt. Im Anschluss an das Befestigungsende ist die Biegefeder 54 frei bewegbar bzw. elastisch verformbar. Das freie Ende 55 mit dem Kopplungselement 41 ist radial zur Schwenkachse S bewegbar. In der Entkopplungsstellung D liegt das Kopplungselement 41 der Kopplungsaussparung 42 rechtwinklig und vorzugsweise radial zur Schwenkachse S gegenüber, ohne in die Kopplungsaussparung 42 einzugreifen. Die Biegefeder 54 erstreckt sich bogenförmig um die Schwenkachse S, beispielsweise in einem Winkelbereich von mindestens 90 Grad oder mindesten 120 Grad oder mindestens 180 Grad. Die Biegefeder 54 ist parallel zu einer Ebene angeordnet, die rechtwinklig zur Schwenkachse S orientiert ist.

Das freie Ende 55 der Biegefeder 54 ist der zweiten Branche 23 zugewandt und für die zweite Branche 23 zugänglich. Um bei einer Schließbewegung in Richtung der Schließstellung II in Kontakt mit dem freien Ende 55 gelangen zu können, kann an der zweiten Branche 23 ein Vorsprung 60 vorhanden sein, der in Richtung der ersten Branche 22 bzw. der Schwenkachse S von der zweiten Branche 23 weg ragt. Er ist dazu eingerichtet, die Biegefeder 54 im Bereich des freien Endes 55 zu beaufschlagen und in der Schließstellung II der Branchen 22, 23 die Kopplungsstellung C herzustellen (Figur 9).

Die Besonderheit bei dieser Ausführungsform gemäß der Figuren 8 und 9 besteht darin, dass die Vorspanneinrichtung 49 eine Baueinheit bildet mit dem Kopplungselement 41, vorzugsweise eine integrale Baueinheit. Die Funktionsweise entspricht ansonsten den bisher beschriebenen Ausführungsbeispielen, so dass auf die vorstehende Beschreibung Bezug genommen werden kann.

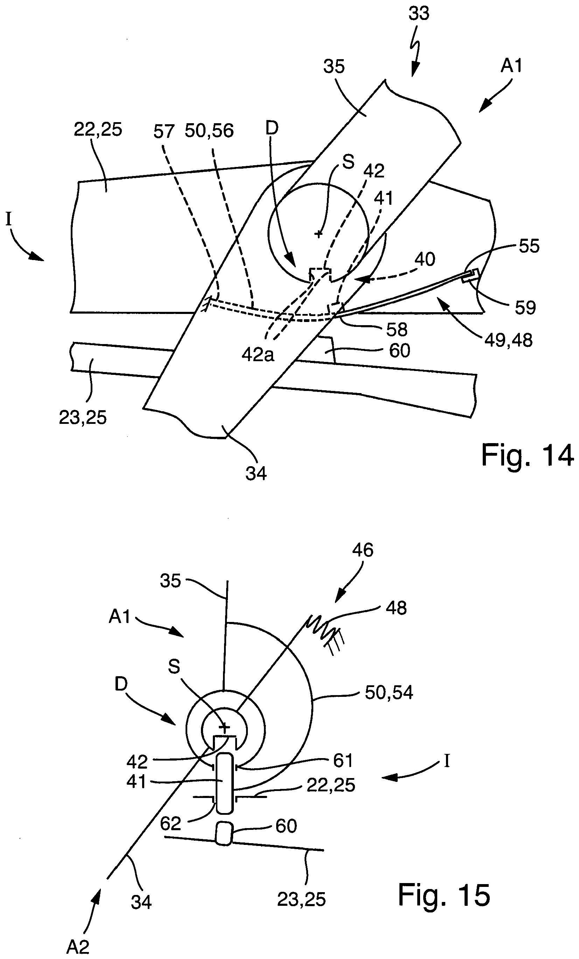

In Figur 15 ist ein gegenüber den Figuren 8 und 9 abgewandeltes Ausführungsbeispiel der Betätigungsvorrichtung 33 veranschaulicht. Das Kopplungselement 41 ist analog zum Ausführungsbeispiel der Figuren 8 und 9 an einer Biegefeder 54 angeordnet, die bei diesem Ausführungsbeispiel am Antriebselement 35 befestigt ist. Das Antriebselement 35 weist eine Aussparung oder ein Lager 61 auf, mittels dem das Kopplungselement 41 gegen die Kraft der Biegefeder 54 in Richtung zur Schwenkachse S hin verschiebbar gelagert ist. Das Lager 61 kann beispielsweise durch ein Durchgangsloch gebildet sein. Das Kopplungselement 41 kann bei dieser Ausführungsform die Gestalt eines Stiftes haben. In Umfangsrichtung um die Schwenkachse S stützt sich das Kopplungselement 41 über das Lager 61 am Antriebselement 35 ab. Die Kopplungsaussparung 42 ist am Betätigungselement 34 vorhanden.

In der Offenstellung I der Branchen 22, 23 nimmt das Kopplungselement 41 die Entkopplungsstellung D ein. Es befindet sich außer Eingriff mit der Kopplungsaussparung 42, so dass das Betätigungselement 34 um die Schwenkachse S bewegt werden kann, ohne eine Bewegung des Antriebselements 35 zu verursachen. Das Betätigungselement 34 kann über die Rückstelleinrichtung 46 und beispielsgemäß das zweite Rückstellelement 48 in die zweite Ausgangslage A2 gedrängt werden. Bei dieser Ausführungsform weist die Rückstelleinrichtung 46 kein Rückstellelement auf, um das Antriebselement 35 in die erste Ausgangslage A1 zu drängen. Das Antriebselement 35 wird in der ersten Ausgangslage A1 gehalten und gegen eine Bewegung um die Schwenkachse S verriegelt bzw. gesichert, beispielsgemäß mittels des Kopplungselements 41. Hierzu greift das Kopplungselement 41, beispielsweise mit seinem von der Kopplungsaussparung 42 abgewandten Ende, in eine Verriegelungsaussparung 62 an der ersten Branche 22 ein.

Wenn die Branchen aus der Offenstellung I in die Schließstellung II bewegt werden, wird das Kopplungselement 41 mittels des Vorsprungs 60 an der zweiten Branche 23 außer Eingriff mit der Verriegelungsaussparung 62 und außerdem in Eingriff mit der Kopplungsaussparung 42 gebracht, so dass das Kopplungselement 41 die Kopplungsstellung C einnimmt (nicht dargestellt). Wie bei den anderen Ausführungsbeispielen bewegen sich das Betätigungselement 34 und das Antriebselement 35 in der Kopplungsstellung C des Kopplungselements 41 gemeinsam um die Schwenkachse S, wenn das Betätigungselement 34 manuell betätigt wird. In diesem Zustand wirkt die Rückstellkraft der Rückstelleinrichtung 46 nicht nur auf das Betätigungselement 34, sondern auch auf das Antriebselement 35.

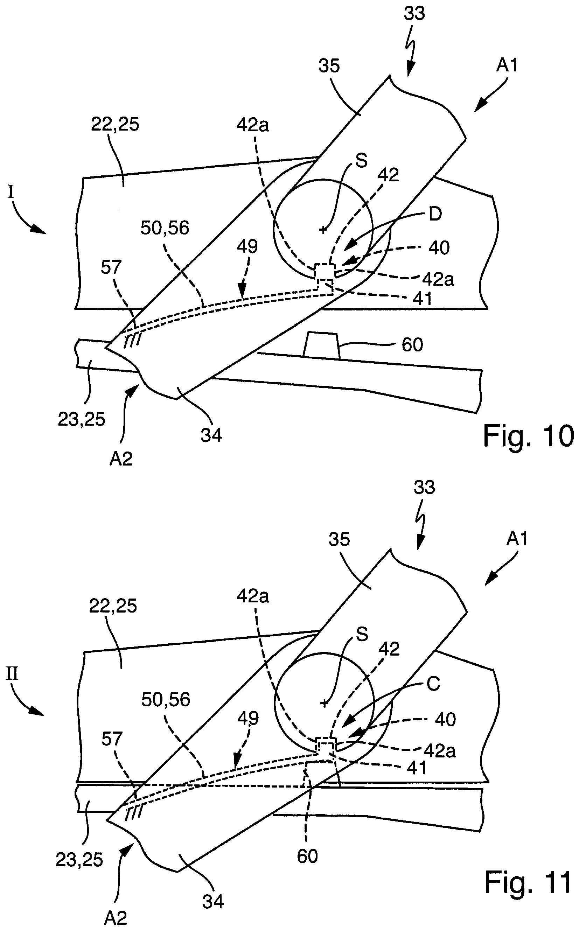

In den Figuren 10 und 11 ist ein weiteres Ausführungsbeispiel der Betätigungsvorrichtung 33 schematisch veranschaulicht. Der wesentliche Unterschied gegenüber dem Ausführungsbeispiel aus den Figuren 8 und 9 besteht darin, dass das Federelement 50 als Blattfeder 56 ausgebildet ist, die sich in ihrem unbeaufschlagten Ausgangszustand in der Entkopplungsstellung D geradlinig oder leicht gekrümmt von einem mit dem Betätigungselement 34 befestigten Befestigungsende 57 zum freien Ende 55 hin erstreckt. Am freien Ende 55 ist das Kopplungselement 41 angeordnet und kann wie beim vorhergehenden Ausführungsbeispiel eine Baueinheit mit der Blattfeder 56 bilden. Zur Bildung der Baueinheit kann das Kopplungselement 41 unlösbar mit der Blattfeder 56 verbunden sein oder integral mit der Blattfeder 56 ausgebildet sein.

Die Funktionsweise zur Herstellung des Kopplungszustandes bzw. der Kopplungsstellung C entspricht den vorhergehenden Ausführungsbeispielen, so dass auf die bevorstehende Beschreibung verwiesen werden kann.

Der Abstand der Blattfeder 56 von der Schwenkachse S nimmt vorzugsweise ausgehend vom freien Ende 55 entlang der Erstreckung der Blattfeder 56 zum Befestigungsende 57 kontinuierlich zu. Eine Gerade, die das Befestigungsende 57 mit dem freien Ende 55 verbindet verläuft versetzt zur Schwenkachse und erstreckt sich beispielsgemäß nicht durch das Antriebselement 35.

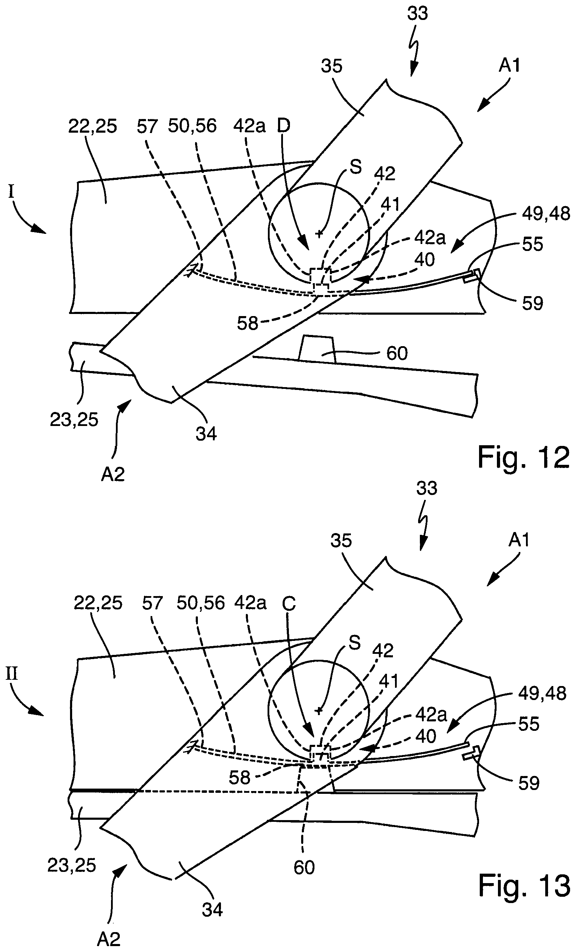

Bei dem in den Figuren 12-14 veranschaulichten Ausführungsbeispiel der Betätigungsvorrichtung 33 ist das Federelement 50 wiederum durch eine Blattfeder 56 gebildet, ähnlich wie beim Ausführungsbeispiel der Figuren 10 und 11. Der wesentliche Unterschied zum vorhergehenden Ausführungsbeispiel besteht darin, dass sich die Blattfeder 56 von dem mit dem Betätigungselement 34 befestigten Befestigungsende 57 bogenförmig gekrümmt bis zu einem freien Ende 55 erstreckt, wobei das Kopplungselement 41 in einem zentralen Abschnitt 58 der Blattfeder 56 zwischen den beiden Enden 55, 57 angeordnet ist.

Das freie Ende 55 stützt sich in der Entkopplungsstellung D an einem Widerlager 59 ab, das am Schaftteil 25 der ersten Branche 22 angeordnet ist. In der Entkopplungsstellung D befindet sich das Kopplungselement 41 vollständig außerhalb der Kopplungsaussparung 42 und liegt dieser rechtwinklig oder radial zur Schwenkachse S gegenüber. Die Krümmung der Blattfeder 56 ist auf der der Schwenkachse S zugewandten Seite konkav und dementsprechend auf der der Schwenkachse S abgewandten Seite konvex. In der Entkopplungsstellung D bildet die Blattfeder 56 der Vorspanneinrichtung 49 gleichzeitig das zweite Rückstellelement 48, um das Betätigungselement 34 in die zweite Ausgangslage A2 zurückzudrängen. Dieser Effekt ist schematisch in Figur 14 veranschaulicht. Wird in der Entkopplungsstellung D das Betätigungselement 34 aus der zweiten Ausgangslage A2 weg geschwenkt, stützt sich die Blattfeder 56 zwischen dem Befestigungsende 57 und dem Widerlager 59 ab und wird gegenüber ihrem Ruhezustand (Figur 12) elastisch verformt und beispielsgemäß gebogen. Dadurch entsteht eine Rückstellwirkung bzw. ein Rückstellmoment um die Schwenkachse S, das auf das Betätigungselement 34 wirkt und dieses in Richtung der zweiten Ausgangslage A2 zurückdrängt. Wie es in Figur 14 zu erkennen ist, kann bei einem aus der zweiten Ausgangslage A2 ausgelenkten Betätigungselement 34 kein Kopplungszustand bzw. keine Kopplungsstellung C hergestellt werden.

Wenn sich das Betätigungselement 34 in der zweiten Ausgangslage A2 und das Antriebselement 35 in der ersten Ausgangslage A1 befindet, ist das Herstellen des Kopplungszustandes bzw. der Kopplungsstellung C ermöglicht. Wenn dann die Branchen 22, 23 aus der Offenstellung I in die Schließstellung II bewegt werden, gelangt das Schaftteil 25 der zweiten Branche 23 und beispielsgemäß der Vorsprung 60 in Kontakt mit dem zentralen Abschnitt 58 der Blattfeder 56 und bewegt das Kopplungselement 41 in die Kopplungsaussparung 42. Die Kopplungsstellung C ist hergestellt. Wie es in Figur 13 zu erkennen ist, gelangt dabei gleichzeitig das freie Ende 55 der Blattfeder 56 außer Eingriff mit dem Widerlager 59. Das freie Ende 55 wird bei Erreichen der Schließstellung II durch den Kontakt mit der zweiten Branche 23 vom Widerlager 59 abgehoben. Dadurch ist erreicht, dass die gemeinsame Bewegung des Betätigungselements 34 mit dem gekoppelten Antriebselement 35 um die Schwenkachse S ungehindert vom Widerlager 59 durchgeführt werden kann.

Ist der Kopplungszustand bzw. die Kopplungsstellung C hergestellt und wird anschließend eine Bewegung des Betätigungselements 34 und des Antriebselements 35 um die Schwenkachse S durchgeführt, wird das Kopplungselement 41, das am Betätigungselement 34 sitz, in der am Antriebselement 35 angeordneten Kopplungsaussparung 42 mit einer Kraft in Umfangsrichtung um die Schwenkachse S beaufschlagt, so dass die Kopplungsstellung C aufrecht erhalten wird. Erst wenn das Antriebselement 35 die erste Ausgangslage A1 und das Betätigungselement 34 die zweite Ausgangslage A2 erreicht hat, kann die Kopplungsstellung C gelöst werden, wenn die Branchen 22, 23 die Offenstellung I einnehmen oder in die Offenstellung I gebracht werden. Außerhalb der Ausgangslagen A1, A2 ist die durch die Vorspanneinrichtung 49 bewirkte Kraft auf das Kopplungselement 41 ausreichend gering, dass aufgrund der Klemmkraft in Umfangsrichtung um die Schwenkachse S außerhalb der Ausgangslagen A1, A2 kein Lösen der Kopplungsstellung C erfolgt.

Die im Zusammenhang mit den Figuren 4 und 5 erwähnte Rückstelleinrichtung 46 kann bei allen Ausführungsbeispielen vorhanden sein, insbesondere den Ausführungsbeispielen gemäß der Figuren 8-11. Beim Ausführungsbeispiel gemäß der Figuren 12-14 ist das zweite Rückstellelement 48 gleichzeitig durch die Vorspanneinrichtung 49 und beispielsgemäß die Blattfeder 56 gebildet. Zusätzlich kann das erste Rückstellelement 47 entsprechend der Figuren 4, 5 vorhanden sein.

Bei allen Ausführungsformen kann am Schaftteil 25 der zweiten Branche 23 ein Vorsprung 60 oder eine andere geeignete Geometrie gebildet sein, so dass in der Schließstellung II das Bewegen des Kopplungselements 41 in die Kopplungsaussparung 42 zur Herstellung der Kopplungsstellung C ermöglicht wird.

Bei allen Ausführungsbeispielen kann das chirurgische Instrument 20 optional als elektrochirurgisches Instrument ausgestaltet sein. Es kann sich beispielsweise um ein monopolares oder ein bipolares elektrochirurgisches Instrument handeln. Hierzu kann das Instrument 20 über eine elektrische Verbindungseinrichtung 65 mit einer elektrischen Leitung verbunden werden (Figuren 1-3). Innerhalb einer der Branchen und beispielsgemäß der zweiten Branche 23, erstreckt sich eine elektrische Leiteranordnung, mittels der das Werkzeug 30 und/oder zumindest eine der Gewebekontaktflächen 27 elektrisch verbunden werden können. Eingeklemmtes Gewebe kann auf diese Weise elektrochirurgisch behandelt werden, beispielsweise zur Gefäßversiegelung.

Die Erfindung betrifft ein chirurgisches Instrument 20 mit einer ersten Branche 22 und einer zweiten Branche 23, die gelenkig aneinander angeordnet sind. Ein Werkzeug 30 ist in einem Werkzeugkanal 31 in einer Längsrichtung L verschiebbar gelagert, vorzugsweise in oder an der ersten Branche 22. Mittels einer Betätigungsvorrichtung 33 kann das Werkzeug 30 in Längsrichtung L bewegt werden. Zur Betätigungsvorrichtung 33 gehört ein Betätigungselement 34 und ein Antriebselement 35, die um eine Schwenkachse S schwenkbar gelagert sind, vorzugsweise an der ersten Branche 22. Über eine Kopplungseinrichtung 40 kann eine drehfeste Kopplung zwischen dem Betätigungselement 34 und dem Antriebselement 35 hergestellt werden. Die drehfeste Kopplung wird bevorzugt dadurch hergestellt, dass die Branchen 22, 23 in eine Schließstellung II bewegt werden.

### Bezugszeichenliste:

- 20: elektrochirurgisches Instrument
- 21: Gelenk
- 22: erste Branche
- 23: zweite Branche
- 24: Maulteil
- 25: Schaftteil
- 26: Griffstück
- 27: Gewebekontaktfläche

- 30: Werkzeug
- 31: Werkzeugkanal
- 32: Messer
- 33: Betätigungsvorrichtung
- 34: Betätigungselement
- 35: Antriebselement
- 36: Übertragungseinrichtung
- 37: Übertragungsglied

- 40: Kopplungseinrichtung
- 41: Kopplungselement
- 42: Kopplungsaussparung

- 46: Rückstelleinrichtung
- 47: erstes Rückstellelement
- 48: zweites Rückstellelement
- 49: Vorspanneinrichtung
- 50: Federelement
- 51: Schraubenfeder
- 52: Ringstufe
- 53: Kopfteil
- 54: Biegefeder
- 55: freies Ende der Biege- oder Blattfeder
- 56: Blattfeder
- 57: Befestigungsende der Blattfeder
- 58: zentraler Abschnitt der Blattfeder
- 59: Widerlager
- 60: Vorsprung
- 61: Lager
- 62: Verriegelungsaussparung

- 65: elektrische Verbindungseinrichtung
- 66: elektrische Leitung

- I: Offenstellung
- II: Schließstellung

- A1: erste Ausgangslage
- A2: zweite Ausgangslage
- C: Kopplungsstellung
- D: Entkopplungsstellung
- L: Längsrichtung
- S: Schwenkachse

## Patentansprüche

1. Chirurgisches Instrument (20) aufweisend:
- eine erste Branche (22) und eine zweite Branche (23), die gelenkig aneinander gelagert sind und zwischen wenigstens einer Offenstellung (I) und einer Schließstellung (II) relativ zueinander schwenkbar sind,
- ein Werkzeug (30), das an oder in der ersten Branche (22) in einer Längsrichtung (L) geführt bewegbar gelagert ist,
- ein Betätigungselement (34), und ein Antriebselement (35), die schwenkbar um eine Schwenkachse (S) an der ersten Branche (22) gelagert sind, wobei der Antriebselement (35) mit dem Werkzeug (30) bewegungsgekoppelt ist,
- eine Kopplungseinrichtung (40) aufweisend ein Kopplungselement (41), das zwischen einer Kopplungsstellung (C) und einer Entkopplungsstellung (D) bewegbar ist **dadurch gekennzeichnet,**
**dass** das Kopplungselement (41) dazu eingerichtet ist, in der Kopplungsstellung (C) eine Drehkopplung zwischen dem Betätigungselement (34) und dem Antriebselement (35) herzustellen und in der Entkopplungsstellung (D) eine Relativdrehung zwischen dem Betätigungselement (34) und dem Antriebselement (35) zuzulassen.

2. Chirurgisches Instrument nach Anspruch 1, wobei das Kopplungselement (41) durch die zweite Branche (22) bewegbar ist.

3. Chirurgisches Instrument nach Anspruch 1 oder 2, wobei die zweite Branche (23) dazu eingerichtet ist, das Kopplungselement (41) aus der Entkopplungsstellung (D) in die Kopplungsstellung (C) zu bewegen, wenn die Branchen (22, 23) relativ zueinander in die Schließstellung (II) geschwenkt werden.

4. Chirurgisches Instrument nach einem der vorhergehenden Ansprüche, wobei das Antriebselement (35) eine Kopplungsaussparung (42) aufweist, in das das Kopplungselement (41) in der Kopplungsstellung (C) hineinragt.

5. Chirurgisches Instrument nach einem der vorhergehenden Ansprüche, wobei das Kopplungselement (41) zwischen der Kopplungsstellung (C) und der Entkopplungsstellung (D) verschiebbar ist.

6. Chirurgisches Instrument nach einem der Ansprüche 1 bis 4, wobei das Kopplungselement (41) zwischen der Kopplungsstellung (C) und der Entkopplungsstellung (D) schwenkbar ist.

7. Chirurgisches Instrument nach einem der vorhergehenden Ansprüche, wobei eine Vorspanneinrichtung (49) vorhanden ist, die dazu eingerichtet ist, eine Vorspannkraft auf das Kopplungselement (41) zu erzeugen, die das Kopplungselement (41) in die Entkopplungsstellung (D) drängt.

8. Chirurgisches Instrument nach Anspruch 7, wobei die Vorspanneinrichtung (49) ein die Vorspannkraft erzeugendes Federelement (50) aufweist.

9. Chirurgisches Instrument nach Anspruch 8, wobei das Federelement (50) und das Kopplungselement (41) miteinander verbunden und relativ zueinander unbeweglich sind.

10. Chirurgisches Instrument nach Anspruch 9, wobei das Federelement (50) und das Kopplungselement (41) integral ausgebildet sind.

11. Chirurgisches Instrument nach einem der Ansprüche 8 bis 10, wobei das Federelement (50) eine Biegefeder (54) ist, die sich bogenförmig um die Schwenkachse (S) erstreckt, oder eine Blattfeder (56) ist.

12. Chirurgisches Instrument nach einem der Ansprüche 8 bis 10, wobei das Federelement (50) eine Schraubenfeder (51) ist.

13. Chirurgisches Instrument nach einem der vorhergehenden Ansprüche, wobei eine Rückstelleinrichtung (46) vorhanden ist, die dazu eingerichtet ist, das Betätigungselement (34) und/oder das Antriebselement (35) in eine Ausgangslage (A1, A2) zu drängen.

14. Chirurgisches Instrument nach Anspruch 13, wobei die Rückstelleinrichtung (46) ein erstes Rückstellelement (47) aufweist, das das Antriebselement (35) in die Ausgangslage (A1) drängt, und wobei ein zweites Rückstellelement (48) aufweist, das das Betätigungselement (34) in die Ausgangslage (A2) drängt.

15. Chirurgisches Instrument nach Anspruch 14, wobei das erste Rückstellelement (47) durch ein elastisch verformbares Übertragungsglied (37) gebildet ist, das zwischen dem Antriebselement (35) und dem Werkzeug (30) angeordnet ist.

## Claims

1. Surgical instrument (20) comprising:
- a first jaw (22) and a second jaw (23) that are supported on one another in a hinged manner and that can be pivoted between an open position (I) and a closed position (II) relative to each other,
- a tool (30) that is movably supported in longitudinal direction (L) in a guided manner on or in the first jaw (22),
- an actuation element (34) and a drive element (35) that are pivotably supported around a pivot axis (S) on the first jaw (22), whereby the drive element (35) is movably coupled with the tool (30),
- a coupling device (40) comprising a coupling element (41) that can be moved between a coupling position (C) and a decoupling position (D)
**characterized in that** the coupling element (41) is configured to establish a rotational coupling between the actuation element (34) and the drive element (35) in the coupling position (C) and to allow a relative rotation between the actuation element (34) and the drive element (35) in the decoupling position (D).

2. Surgical instrument according to claim 1, wherein the coupling element (41) is movable by means of the second jaw (22).

3. Surgical instrument according to claim 1 or 2, wherein the second jaw (23) is configured to move the coupling element (41) out of the decoupling position (D) into the coupling position (C), if the jaws (22, 23) are pivoted relative to one another into the closed position (II).

4. Surgical instrument according to any of the preceding claims, wherein the drive element (35) comprises a coupling recess (42) into which the coupling element (41) projects in the coupling position (C).

5. Surgical instrument according to any of the preceding claims, wherein the coupling element (41) can be shifted between the coupling position (C) and the decoupling position (D).

6. Surgical instrument according to any of the claims 1 to 4, wherein the coupling element (41) can be pivoted between the coupling position (C) and the decoupling position (D).

7. Surgical instrument according to any of the preceding claims, wherein a biasing device (49) is present configured to create a biasing force on the coupling element (41) that urges the coupling element (41) into the decoupling position (D).

8. Surgical instrument according to claim 7, wherein the biasing device (49) comprises a spring element (50) creating the biasing force.

9. Surgical instrument according to claim 8, wherein the spring element (50) and the coupling element (41) are connected with each other and are immovably relative to each other.

10. Surgical instrument according to claim 9, wherein the spring element (50) and the coupling element (41) are integrally formed.

11. Surgical instrument according to any of the claims 8 to 10, wherein the spring element (50) is a flexible spring (54) that extends in an arc-shaped manner around pivot axis (S) or wherein the spring element (50) is a leaf spring (56).

12. Surgical instrument according to any of the claims 8 to 10, wherein the spring element (50) is a helical spring (51).

13. Surgical instrument according to any of the preceding claims, wherein a resetting device (46) is provided that is configured to urge the actuation element (34) and/or the drive element (35) in an initial position (A1, A2).

14. Surgical instrument according to claim 13, wherein the resetting device (46) comprises a first resetting element (47) that urges the drive element (35) into the initial position (A1) and comprises a second resetting element (48) that urges the actuation element (34) into the initial position (A2).

15. Surgical instrument according to claim 14, wherein the first resetting element (47) is formed by an elastically deformable transmission element (37) arranged between the drive element (35) and the tool (30).

## Revendications

1. Instrument chirurgical (20), présentant :
- une première branche (22) et une deuxième branche (23) qui sont montées de manière articulée l'une sur l'autre et peuvent être pivotées l'une par rapport à l'autre entre au moins une position d'ouverture (I) et une position de fermeture (II),
- un outil (30) qui est monté sur ou dans la première branche (22), en étant guidé avec possibilité de déplacement dans une direction longitudinale (L),
- un élément d'actionnement (34) et un élément d'entraînement (35) qui sont montés sur la première branche (22) avec possibilité de pivotement autour d'un axe de pivotement (S), l'élément d'entraînement (35) étant couplé en termes de mouvement à l'outil (30),
- un dispositif de couplage (40) comportant un élément de couplage (41) qui peut être déplacé entre une position de couplage (C) et une position de découplage (D), **caractérisé en ce que**
l'élément de couplage (41) est conçu pour établir, dans la position de couplage (C), un couplage rotatif entre l'élément d'actionnement (34) et l'élément d'entraînement (35) et pour autoriser, dans la position de découplage (D), une rotation relative entre l'élément d'actionnement (34) et l'élément d'entraînement (35).

2. Instrument chirurgical selon la revendication 1, dans lequel l'élément de couplage (41) peut être déplacé par la deuxième branche (22).

3. Instrument chirurgical selon la revendication 1 ou 2, dans lequel la deuxième branche (23) est conçue pour déplacer l'élément de couplage (41) de la position de découplage (D) à la position de couplage (C) lorsque les branches (22, 23) sont pivotées l'une par rapport à l'autre pour occuper la position de fermeture (II).

4. Instrument chirurgical selon l'une des revendications précédentes, dans lequel l'élément d'entraînement (35) présente un évidement de couplage (42) dans lequel avance l'élément de couplage (41), dans la position de couplage (C).

5. Instrument chirurgical selon l'une des revendications précédentes, dans lequel l'élément de couplage (41) peut être déplacé par glissement entre la position de couplage (C) et la position de découplage (D).

6. Instrument chirurgical selon l'une des revendications 1 à 4, dans lequel l'élément de couplage (41) peut être pivoté entre la position de couplage (C) et la position de découplage (D).

7. Instrument chirurgical selon l'une des revendications précédentes, dans lequel il est prévu un dispositif de précontrainte (49) qui est conçu pour exercer sur l'élément de couplage (41) une force de précontrainte qui pousse l'élément de couplage (41) dans la position de découplage (D).

8. Instrument chirurgical selon la revendication 7, dans lequel le dispositif de précontrainte (49) présente un élément formant ressort (50) générant la force de précontrainte.

9. Instrument chirurgical selon la revendication 8, dans lequel l'élément formant ressort (50) et l'élément de couplage (41) sont reliés l'un à l'autre et ne peuvent pas être déplacés l'un par rapport à l'autre.

10. Instrument chirurgical selon la revendication 9, dans lequel l'élément formant ressort (50) et l'élément de couplage (41) sont réalisés d'une seule pièce.

11. Instrument chirurgical selon l'une des revendications 8 à 10, dans lequel l'élément formant ressort (50) est un ressort de flexion (54) qui s'étend sous forme d'arc autour de l'axe de pivotement (S), ou est un ressort à lame (56).

12. Instrument chirurgical selon l'une des revendications 8 à 10, dans lequel l'élément formant ressort (50) est un ressort hélicoïdal (51).

13. Instrument chirurgical selon l'une des revendications précédentes, dans lequel il est prévu un dispositif de rappel (46) qui est conçu pour pousser l'élément d' actionnement (34) et/ou l'élément d' entraînement (35) dans une position initiale (A1, A2).

14. Instrument chirurgical selon la revendication 13, dans lequel le dispositif de rappel (46) présente un premier élément de rappel (47), qui pousse l'élément d'entraînement (35) dans la position initiale (A1), et présente un deuxième élément de rappel (48) qui pousse l'élément d'actionnement (34) dans la position initiale (A2).

15. Instrument chirurgical selon la revendication 14, dans lequel le premier élément de rappel (47) est constitué d'un organe de transmission (37) déformable élastiquement, qui est disposé entre l'élément d'entraînement (35) et l'outil (30).
